(19) Europäisches Patentamt

European Patent Office

Office européen des brevets

(11) **EP 0 849 281 B1**

(12) **EUROPEAN PATENT SPECIFICATION**

(45) Date of publication and mention
of the grant of the patent:
**07.05.2003 Bulletin 2003/19**

(51) Int Cl.⁷: **C08B 37/04**, A61L 15/28,
A61L 27/00, A61L 31/00,
A61L 15/64

(21) Application number: **97310395.5**

(22) Date of filing: **19.12.1997**

(54) **Bioresorbable alginate derivatives**

Bioresorbierbare Alginatderivate

Dérivés d'alginate biorésorbables

(84) Designated Contracting States:
**AT BE CH DE ES FR GB IE IT LI LU NL PT SE**

(30) Priority: **20.12.1996 GB 9626466**

(43) Date of publication of application:
**24.06.1998 Bulletin 1998/26**

(73) Proprietor: **Johnson & Johnson Medical Ltd.
Edinburgh EH2 4NH (GB)**

(72) Inventors:
• **Doyle, Peter J.
Tullibody, Clackmannanshire, FK10 2XD (GB)**
• **Harvey, Wilson
Gargunnock, Stirling, FK8 3AX (GB)**

• **Stilwell, Reginald L.
Arlington, Texas 76018 (US)**

(74) Representative: **Fisher, Adrian John et al
CARPMAELS & RANSFORD
43 Bloomsbury Square
London WC1A 2RA (GB)**

(56) References cited:
**EP-A- 0 693 291      EP-A- 0 815 879
US-A- 3 784 475**

• **DATABASE WPI Week 7520 Derwent
Publications Ltd., London, GB; AN 75-33302w
XP002059181 & JP 50 016 789 A (SAKAI
CHEMICAL IND KK) , 21 February 1975**

**Description**

The present invention relates to processes for the production of bioresorbable alginate derivatives. The present invention also relates to the use of such bioresorbable alginate derivatives in pharmaceutical compositions.

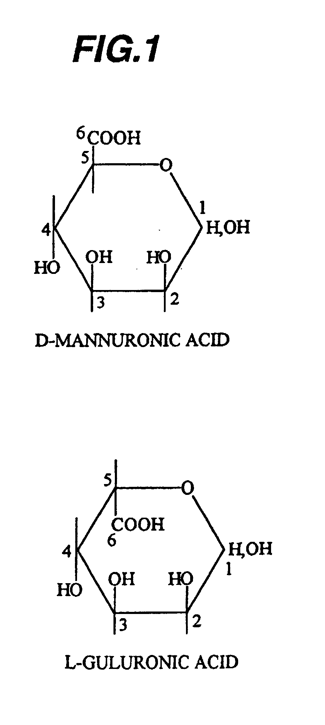

Alginates are linear binary copolymers of D-mannuronic acid (MA) and L-guluronic acid (GA) having the structures shown in Figure 1. The polymers are built up by ether linkages joining the 1- and 4- positions of the MA and GA saccharide residues. Alginates are isolated from marine brown algae, and such naturally occurring alginates generally comprise blocks of MA rich units, GA rich units and mixed sequences of MA and GA units. Commercially available alginates of this type typically contain about 45% of MA.

Alginates are available in the form of alginic acid, various salts, and various ester derivatives such as propyleneglycol alginates. Alginate salts with monovalent cations such as sodium are generally soluble in water. Alginate salts formed with divalent or trivalent cations such as calcium or zinc are generally insoluble in water. The solubility of alginate compositions can thus be controlled over a wide range by varying the sodium/calcium ratio of a mixed sodium/calcium alginate salt. Commercially available alginate products are generally formed from such mixed salts.

Alginate products have long been used in the field of wound healing, especially as a packing material for cavity wounds or for treatment of burns. Alginate materials are sold under the registered trade marks KALTOSTAT (Britcaire Limited), SORBSAN (Pharma-Plast Limited) and ALGOSTERIL (Johnson & Johnson). These products are available in a number of forms, including ropes and pads. These materials are highly absorbent, biocompatible and cheap.

Although alginates have good properties for treating cavity wounds and burns, care has to be taken when changing the dressings to ensure that nothing is left in the wound. Alginate is not bioresorbable, but does tend to fragment. If left in the wound, fragments of alginate will result in the formation of granulomas. It is therefore necessary to rinse the wound out thoroughly with saline solution to ensure that no residual alginate remains.

Alginates have also been shown to have excellent haemostatic properties, but because they are not resorbable they must be removed prior to closure of a wound, which inevitably limits their usefulness in this application.

Accordingly, there exists a need for improved materials, in particular for wound dressing and haemostatic applications, that exhibit the advantages of alginates and are also bioresorbable.

It has long been known that cellulose can be rendered bioresorbable by exposure to an oxidizing agent such as dinitrogen tetroxide, as described in US-A-3122479. The resulting oxidized regenerated cellulose (ORC) is available in the form of a knitted fabric under the registered trade mark SURGICEL for use as an absorbable haemostat. ORC is also available under the registered trade mark INTERCEED for use as an adhesion barrier. The bioresorbable character of ORC is thought to be due to oxidation of the primary hydroxyl groups on the cellulose residues to carboxylate groups.

US-A-4543410 describes absorbent, coherent, flexible structures in the form of fibrous webs and porous sponges comprising water-insoluble, ring oxidized cellulosic bases. It is stated that ring oxidation of the cellulosic bases can selectively convert the hydroxyl groups at the 2, 3 and 6 positions of the anhydroglucose units of cellulose into carboxyl groups, depending on the specific oxidant used. It is stated that dinitrogen tetroxide converts the hydroxyl group at the 6 position into a carboxyl group to produce a mono-carboxyl form of the base (as in the formation of ORC). Periodic acid will open the ring between the 2 and 3 position and convert the hydroxyl groups at the 2 and 3 position into aldehyde groups. The resulting dioxide can be further oxidized with chlorine or dinitrogen tetroxide to product a dicarboxyl or tricarboxyl form of the base. It is stated that ring oxidized cellulosic base sponges having a carboxyl content due to ring oxidation greater than about 15% are haemostatic and bioresorbable. Ring oxidation of starch is also disclosed.

EP-A-0 693 291 discloses a composition comprising a hydrolyzable wound treatment including an oxidised polysaccharide which is cross-linked and has a chemically induced charge and delivery vehicle, the modified polysaccharide and delivery vehicle being in a condition in which the oxidized polysaccharide will not substantially hydrolize prior to application to a wound site.

It has now been found that ring oxidation of alginates with dinitrogen tetroxide results in bioresorbable, oxidized alginate derivatives. This result is surprising, since the saccharide residues making up the alginate molecules are already fully oxidized to carboxylate at the 6 position before treatment with the dinitrogen tetroxide.

Accordingly, the present invention provides a process to prepare an oxidized alginate comprising the steps of: contacting an alginate with an oxidizing agent comprising $N_2O_4$ in an inert solvent to produce said oxidized alginates; followed by isolating and washing the oxidized alginate.

Preferably, at least part of the MA and/or GA saccharide residues of the alginate have been oxidized at the 2- or 3- position. Such oxidation could take place without ring opening, by oxidation of the secondary alcohol groups to keto groups, or it can take place with ring opening to dialdehyde or dicarboxylate derivatives. More preferably, at least 0.2% of the saccharide residues of the alginate have been oxidized at the 2- or 3- position, and still more preferably at least 1.0% of the saccharide residues have been so oxidized.

Preferably, the ring oxidation of the alginate residues has taken place with ring opening to dicarboxylic acid derivatives. As a result, the oxidized alginate according to the present invention preferably has a carboxylate content greater than that of the starting alginic acid. Preferably, the carboxylate content is increased by at least 1%, and more preferably there is at least 2% increase in the number of carboxylate groups relative to the corresponding unoxidized material. The carboxylate content is determined as follows:-

A sample of oxidized alginate (approximately 0.2g) is dissolved in 0.5M sodium hydroxide (5ml) and a couple of drops of 0.1% phenolphthalein indicator solution are added. The excess sodium hydroxide is back-titrated with 0.1M HCl to the phenolphthalein end point (red to clear). A blank value is determined by titrating 5ml 0.1M sodium hydroxide with 0.1M HCl. The value for carboxyl content (percentage by weight) is calculated using the equation:

$$C = \frac{4.5 \times (B\text{-}S) \times M}{W}$$

wherein:

C = percent carboxyl content
B = volume of standard HCl to titrate blank (ml)
S = volume of standard HCl to titrate sample (ml)
M = molality of standard HCl
W = dry weight of sample (g)
(4.5 = milliequivalent weight of carboxyl x 100)

The oxidized alginate is more bioabsorbable and bioassimilable in the mammalian body. Preferably, the oxidized alginate is fully absorbable when implanted in the mammalian body.

The oxidized alginate derivatives have substantially similar solubility behaviour to naturally occurring alginates. In particular, the solubility of oxidized alginate salts can be varied by varying the ratio of sodium and calcium cations. Preferably, the oxidized alginates are substantially insoluble in water. This implies that the oxidized alginates are preferably a salt of the oxidized alginate and divalent or trivalent cations, such as calcium or zinc ions.

Preferably, the oxidized alginates have a weight average molecular weight in the range 10,000 to 1,000,000, more preferably 50,000 to 400,000.

The present invention also provides a pharmaceutical composition comprising an oxidized alginate obtainable by a process according to the invention. The oxidized alginate may be used for the preparation of a wound dressing, surgical implant or prosthesis.

The present invention provides a process to prepare an oxidized alginate comprising the steps of: contacting an alginate with an oxidizing agent comprising dinitrogen tetroxide in an inert solvent to oxidize the alginate; followed by isolating and washing the oxidized alginate. Preferably, the alginate is solid before, during and after the contacting step. For example, the starting alginate may be a calcium alginate foam, web or fleece.

Specific embodiments of the present invention will now be described further, by way of example, with reference to the accompanying drawings, in which:-

Figure 1 shows the structures of the mannuronic acid and guluronic acid building blocks of alginate prior to oxidation;
Figure 2 shows an ion-exchange chromatogram of breakdown products of oxidized alginic acid incubated in serum for 48 hours; and
Figure 3 shows a comparative ion-exchange chromatogram of unoxidized alginic acid incubated in serum for 48 hours.

Example 1

An oxidized alginic acid derivative was prepared as follows.

20 grams of alginic acid (Sigma Chemical Company) was suspended in 150 grams of fluorocarbon solvent (solvent FC77 supplied by 3M Corporation). 20 grams of liquid dinitrogen tetroxide was dissolved carefully in 50 grams of the same fluorinated solvent and slowly added to the alginate suspension over 2 hours at room temperature. The mixture was left to react at room temperature for a further 4 hours. The resulting slurry was decanted into a Buchner funnel fitted with a porous glass frit, and the oxidized alginate was collected. The oxidized alginic acid was washed by reslurrying in fluorocarbon solvent for 10 minutes and collected by Buchner filtration. The oxidized alginic acid was then washed 4 times in 90% isopropanol and twice in 100% isopropanol before being allowed to dry in air.

The oxidized alginic acid was converted to its sodium salt by reacting with 12 grams of sodium acetate dissolved in 200 ml distilled water. The sodium salt of the oxidized alginic acid was reprecipitated as the calcium salt by adding an excess of calcium chloride to the oxidized sodium alginate solution. The calcium alginate was collected by centrifugation, washed extensively in distilled water, and air dried.

The resulting material is an off-white powder that is soluble in 0.5 molar sodium hydroxide solution.

Example 2

The breakdown of the oxidized alginic acid prepared in Example 1 on incubation in serum is studied as follows.

A sample of oxidized alginic acid prepared and described in Example 1 was added to serum at a concentration of 10 mg/ml, and the pH was readjusted to 7.4 using 0.5M NaOH. The dispersion was then incubated at 37°C for 48 hours. Following incubation, the sample was passed through a 0.2 µm filter. A 10 ml sample of the filtrate solution was then injected into a Dionex (registered trade mark) 500 ion-exchange chromatography system fitted with a Carbopac VA 1 an ion exchange column (25 cm x 4 mm) with Carbopac PA 1 guard column (5 cm x 4 mm). The mobile phase was as follows: eluent A - ultrapure water; eluent B - 200 mN sodium hydroxide; and eluent C - 2 M sodium acetate.

The solution gradient programme was as follows:-

initial-100% B; 0-20 minutes - 86% A, 10% B, 4% C; 20-70 minutes - from 86% A, 10% B, 4% C to 40% A, 10% B, 50% C; 70-80 minutes - 100% B.

The sample was injected 15 minutes into the run, and data was collected from the moment the sample was injected until the end of the run.

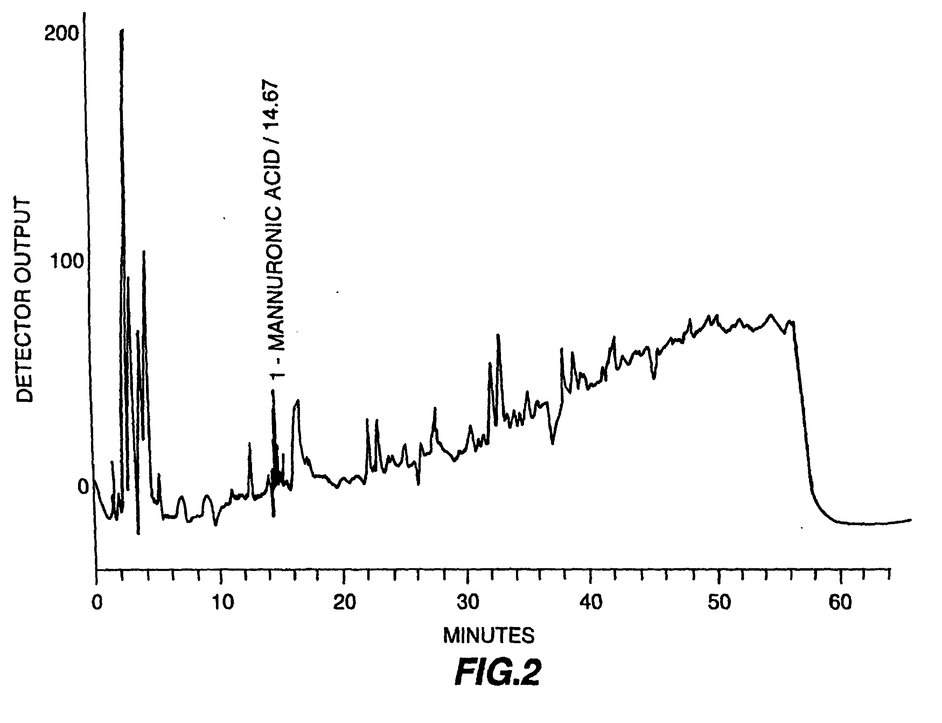

The chromatogram shown in Figure 2 exhibits a number of elution peaks between 10 minutes and 50 minutes, corresponding to various fragments of the oxidized alginic acid that has undergone breakdown in the serum. The peak corresponding to mannuronic acid (identified in a comparative run with added pure mannuronic acid) is marked.

This example illustrates that oxidized alginic acid undergoes breakdown into a number of soluble components in serum at 37°C.

Example 3 (Comparative)

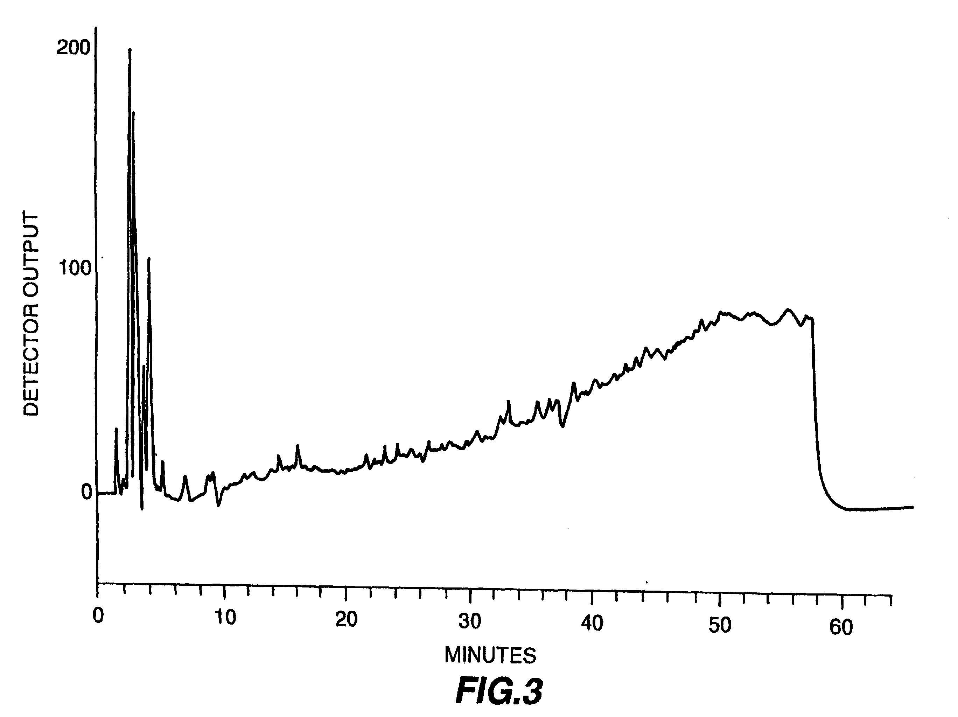

The experimental procedure of Example 2 was repeated exactly with unoxidized alginic acid in place of the oxidized alginic acid. The resulting chromatogram is shown in Figure 3. It can be seen that the chromatogram for alginic acid is substantially free of the elution peaks for breakdown products observed for the oxidized alginic acid. This accords with clinical observations that unoxidized alginates do not undergo breakdown into soluble components *in vivo.*

Example 4

Samples of oxidized alginic acid were prepared as described in Example 1, but with varying oxidation times in the dimitrogen tetroxide solution. The carboxylate content of the oxidized alginic acids was then determined by titration, as described above. The results were as follows:-

| Oxidation Time (hrs) | Carboxylate Content (wt.%) |
|---|---|
| 0 (comparative) | 23.12 |
| 2 | 23.15 |
| 4 | 23.51 |
| 20 | 24.90 |
| 72 | 27.25 |

It can thus be seen that the extent of oxidation to form new carboxylate groups increases with time. It can also be seen that relatively few additional carboxylate groups, e.g. about 1.5% based on the original carboxylate groups, are needed to produce the bioabsorbable alginate after four hours.

Example 5

The properties of oxidized alginic acid prepared as described in Example 1 were studied by thermogravimetry and differential scanning calorimetry (DSC) in air from 30°C to 200°C at 10°C per minute. The TGA results were as follows:-

| Oxidation Time (hrs) | Weight Loss (%) |
|---|---|
| 0 (comparative) | 16.98% |

4

(continued)

| Oxidation Time (hrs) | Weight Loss (%) |
|---|---|
| 20 | 25.09% |
| 72 | 38.31% |

In addition, the oxidized samples showed a marked endotherm above 100°C and changed colour to black.

The above embodiments have been described by way of example only. Many other embodiments falling within the scope of the accompanying claims will be apparent to the skilled reader.

### Example 6

The properties of oxidised alginate *in vivo* were studied as follows:-

(1) Preparation of Comparative Alginate Pads.
Calcium alginate (0.5g) and sodium alginate (1g) were homogenised in 100ml distilled water and poured into a 10cm x 10cm petri dish. The solution was frozen, freeze dried and the resultant pad cut into I x 0.5cm blocks. These were γ-irradiation sterilised.

(2) Preparation of Oxidised Alginate Pads.
Oxidised calcium alginate (0.5g) and oxidised sodium alginate (1g) prepared as described in Example 1 were homogenised in 100ml distilled water and poured into a 10cm x 10cm petri dish. The solution was frozen, freeze dried and the resultant pad cut into 1 x 0.5cm blocks. These were γ-irradiation sterilised.

(3) *In Vivo* study

Twelve Sprague Dawley rats were used in the study. Under standard operating conditions, two pads of each material were subcutaneously implanted on the ventral surface of each rat. Four rats were sacrificed on 3, 7 and 14 days post-implantation. The alginate pads were removed at that time complete with the overlying dermis and the underlying musculature. The samples were fixed in formaldehyde and processed for routine histology. Sections of the alginate pads were made from as close to the centre of the pads as possible. These were stained with either H&E or Masson's Trichrome and examined under a light microscope. The number of neutrophils present in each section was used as an indicator of the intensity of the inflammatory reaction.

The oxidised alginate pads were found to resorb at a faster rate than those prepared from normal alginate. The oxidised alginate pads were also found to elicit a reduced inflammatory response when compared to the normal material..

## Claims

1. A process to prepare an oxidized alginate comprising the steps of: contacting an alginate with an oxidizing agent comprising $N_2O_4$ in an inert solvent to produce said oxidized alginates; followed by isolating and washing the oxidized alginate.

2. A process according to claim 1, wherein the alginate is solid before, during and after said contacting step.

3. A process according to claim 1, wherein at least part of the saccharide residues of the alginate have been oxidized at the 2- or 3- position.

4. A process according to claim 3, wherein at least 0.1% of the saccharide residues of the alginate have been oxidized at the 2- or 3- position.

5. A process according to claim 4, wherein at least 1.0% of the saccharide residues of the alginate have been oxidized at the 2- or 3- position.

6. A process according to any preceding claim, wherein said oxidation has taken place with ring opening.

7. A process according to any preceding claim, wherein the carboxylate content is increased by at least 1% relative to the corresponding unoxidized alginate.

8. A process according to any preceding claim wherein the oxdized alginate has a weight average molecular weight in the range 10,000 to 1,000,000.

9. A pharmaceutical composition comprising an oxidized alginate obtainable by a process comprising the steps of: contacting an alginate with an oxidizing agent comprising $N_2O_4$ in an inert solvent to produce said oxidized alginates; followed by isolating and washing the oxidized alginate.

**Patentansprüche**

1. Verfahren zum Herstellen eines oxidierten Alginats, umfassend die Schritte: Kontaktieren eines Alginats mit einem oxidierenden Agens, umfassend $N_2O_4$, in einem inerten Lösungsmittel, um die oxidierten Alginate herzustellen; gefolgt vom Isolieren und Waschen der oxidierten Alginate.

2. Verfahren nach Anspruch 1, wobei das Alginat vor, während und nach dem Kontaktierungsschritt fest ist.

3. Verfahren nach Anspruch 1, wobei mindestens ein Teil der Saccharidreste des Alginats an der 2- oder 3-Position oxidiert worden ist.

4. Verfahren nach Anspruch 3, wobei mindestens 0,1% der Sacchardireste des Alginats an der 2- oder 3-Position oxidiert worden sind.

5. Verfahren nach Anspruch 4, wobei mindestens 1,0% des Sacchardidreste des Alginats an der 2- oder 3-Position oxidiert worden ist.

6. Verfahren nach einem der vorangehenden Ansprüche, wobei die Oxidation mit einer Ringöffnung stattgefunden hat.

7. Verfahren nach einem der vorangehenden Ansprüche, wobei der Carboxylatgehalt um mindestens 1%, relativ zum entsprechenden nicht-oxidierten Alginat, angestiegen ist.

8. Verfahren nach einem der vorangehenden Ansprüche, wobei das oxidierte Alginat ein durchschnittliches Molekulargewicht im Bereich von 10.000 bis 1.000.000 hat.

9. Pharmazeutische Zusammensetzung, umfassend ein oxidiertes Alginat, erhältlich durch ein Verfahren, das die Schritte umfaßt: Kontaktieren eines Alginats mit einem oxidierenden Agens, umfassend $N_2O_4$, in einem inerten Lösungsmittel, um die oxidierten Alginate zu erzeugen; gefolgt vom Isolieren und Waschen des oxidierten Alginats.

**Revendications**

1. Procédé pour préparer un alginate oxydé comprenant les étapes consistant à : mettre en contact un alginate avec un agent oxydant comprenant $N_2O_4$ dans un solvant inerte pour produire lesdits alginates oxydés; puis isoler et laver l'alginate oxydé.

2. Procédé selon la revendication 1, dans lequel l'alginate est solide avant, pendant et après ladite étape de mise en contact.

3. Procédé selon la revendication I, dans lequel au moins une partie des résidus saccharide de l'alginate ont été oxydés en position 2 ou 3.

4. Procédé selon la revendication 3, dans lequel au moins 0,1% des résidus saccharide de l'alginate ont été oxydés en position 2 ou 3.

5. Procédé selon la revendication 4, dans lequel au moins 1,0% des résidus saccharide de l'alginate ont été oxydés en position 2 ou 3.

6. Procédé selon l'une quelconque des revendications précédentes, dans lequel ladite oxydation a eu lieu avec une ouverture de cycle.

7. Procédé selon l'une quelconque des revendications précédentes, dans lequel la teneur en carboxylate est accrue d'au moins 1% par rapport à l'alginate non oxydé correspondant.

8. Procédé selon l'une quelconque des revendications précédentes, dans lequel l'alginate oxydé a une masse moléculaire moyenne en poids dans la gamme de 10 000 à 1 000 000.

9. Composition pharmaceutique comprenant un alginate oxydé susceptible d'être obtenu par un procédé comprenant les étapes consistant à : mettre en contact un alginate avec un agent oxydant comprenant $N_2O_4$ dans un solvant inerte pour produire lesdits alginates oxydés; puis isoler et laver l'alginate oxydé.

# FIG.1

D-MANNURONIC ACID

L-GULURONIC ACID

FIG.2

EP 0 849 281 B1

FIG.3